# EUROPÄISCHE PATENTSCHRIFT

(11) **EP 1 792 923 B1**
(45) Veröffentlichungstag und Bekanntmachung des Hinweises auf die Patenterteilung: **30.03.2011**
(21) Anmeldenummer: 05026265.8
(22) Anmeldetag: 01.12.2005
(51) Int. Cl.: C08F 220/38, A61L 27/14

(54) **Polymerzusammensetzung mit hohem Brechungsindex**
Compostion of poylmers with high refractive index
Composition de polymères avec un haut indice de réfraction

(43) Veröffentlichungstag der Anmeldung: 06.06.2007
(73) Patentinhaber: CORONIS GmbH, 81241 München (DE)
(72) Erfinder: Storsberg, Joachim Dr., 55286 Wörstadt (DE); Laschewski, André Prof. Dr., 14469 Potsdam (DE); Görnitz. Eckhard Dr., 14513 Teltow (DE); Müller-Lierheim, Wolfgang Dr., 81243 München (DE); Winter, Elsbeth, 87700 Memmingen (DE)
(74) Vertreter: Eisenführ, Speiser & Partner

(56) Entgegenhaltungen:
- EP-A- 1 512 704
- PATENT ABSTRACTS OF JAPAN Bd. 018, Nr. 111 (C-1170), 23. Februar 1994 (1994-02-23) & JP 05 304853 A (MASAHIRO OKAMURA), 19. November 1993 (1993-11-19)

## Beschreibung

Die Erfindung betrifft die Verwendung einer Polymerzusammensetzung mit hohem Brechungsindex für ophthalmologische Vorrichtungen.

Erfindungsgemäß werden Polymerzusammensetzungen mit hohem Brechungsindex bereitgestellt, die besonders geeignet sind zur Verwendung für Augenimplantate. Als Augenimplantate sind insbesondere Intraokularlinsen (IOL), Hornhautimplantate, Keratoprothesen usw. anzusehen. Intraokularlinsen sind schon sehr lange bekannt. Sie werden chirurgisch in das Auge eingesetzt und ersetzen die natürliche Linse des Auges, um bei einem Patienten mit getrübter oder beschädigter Linse das Sehvermögen wiederherzustellen. Die natürliche Linse muss ersetzt werden, wenn sie z.B. bei einem Unfall beschädigt wurde oder, was die Regel ist, wenn die Linse durch Katarakt getrübt ist.

Intraokularlinsen können aus harten oder weichen Polymeren hergestellt werden. Harte Polymere haben den Vorteil, dass sie mechanisch stabil und gut zu bearbeiten sind. Sie sind aber schwierig einzusetzen. Die Intraokularlinse wird in der Regel nach einem chirurgischen Einschnitt in das Auge hineingeschoben. Es ist daher wünschenswert, dass die Linse flexibel ist, um den Einschnitt so gering wie möglich halten zu können. Wenn das für die Linse verwendete Polymer so elastisch ist, dass die Linse gefaltet werden kann bzw. wenn das Material so flexibel ist, dass es aufgerollt werden kann, kann der Einschnitt noch weiter verkleinert werden. Dies ist vorteilhaft und wünschenswert, damit die Beschädigung des Auges möglichst gering gehalten wird und die Heilung schneller erfolgt.

Weiche Linsen lassen sich gut einsetzen, ihnen fehlt aber häufig die Formstabilität. Weiterhin müssen Intraokularlinsen solche elastischen Eigenschaften aufweisen, dass sie sich einerseits zusammenfalten oder rollen lassen, andererseits aber nach dem Einsetzen ihre ursprüngliche Form wieder annehmen und diese Form auch behalten. Das Material für Intraokularlinsen darf nicht zu weich sein und darf auch nicht einen Memory-Effekt zeigen. Für Intraokularlinsen geeignete Polymere müssen daher eine Kombination von einander widersprechenden Eigenschaften vereinigen.

Ein weiteres wesentliche Erfordernis für ein in das Auge einzusetzendes Polymer ist ein ausreichend hoher Brechungsindex. Die Wirkung einer optischen Linse ist bei vorgegebener Geometrie umso stärker, je mehr sich der Brechungsindex von dem des umgebenden Mediums unterscheidet. Entsprechend kann eine Linse umso dünner sein, je höher der Brechungsindex des Materials, aus dem sie hergestellt wird, ist. Bekannte Materialien haben in der Regel einen Brechungsindex im Bereich von 1,45 bis 1,56, so dass aus diesen Materialien hergestellte Intraokularlinsen relativ dick sein müssen. Je dicker die Linse jedoch ist, desto schwieriger ist sie einzusetzen. Wünschenswert sind daher transparente Polymere mit einem Brechungsindex (gemessen gegen Vakuum) von mehr als 1,60.

Eine weitere Bedingung für ein zur Herstellung von Augenimplantaten geeignetes Polymer ist die Transparenz. Geeignete Polymere müssen eine hohe Lichtdurchlässigkeit haben, wobei ggf. bestimmte Wellenbereiche herausgefiltert werden können durch Zusatz von geeigneten Inhaltsstoffen.

Auch die Glasübergangstemperatur ist ein zu beachtender Parameter. Sie muss auf jeden Fall unter 37°C liegen, damit die Linsen bei Körpertemperatur verarbeitbar sind. Eine Glasübergangstemperatur im Bereich von weniger als 15°C wird als geeignet und von weniger als 10°C als wünschenswert angesehen.

Acrylate und Methacrylate wurden aufgrund ihrer guten Verträglichkeit schon sehr lange zur Herstellung von Intraokularlinsen und Augenimplantaten verwendet. Homopolymere der üblicherweise verwendeten Monomere haben allerdings ungenügende Festigkeitseigenschaften und ihr Brechungsindex ist in der Regel zu gering. Es wurde daher versucht, diese Eigenschaften durch Copolymerisation zu verbessern. Hierzu gibt es sehr viele unterschiedliche Ansätze.

Um die Brechkraft zu erhöhen, wurde vorgeschlagen, phenylhaltige Acrylate und Methacrylate einzusetzen. Auch Kombinationen aus hydrophoben und hydrophilen Anteilen wurden verwendet, um dem Material günstige Eigenschaften zu verleihen. So ist z.B. aus EP 0 898 972 ein Material für weiche Intraokularlinsen bekannt, das durch Polymerisation eines hydrophilen Monomers mit einem aromatischen (Meth)acrylat, einem Alkyl(meth)acrylat und einem vernetzbaren Monomer erhalten wird. Das hydrophile Monomer kann beispielsweise (Meth)acrylamid sein, das aromatische (Meth)acrylat kann beispielsweise Phenyloxyethylacrylat sein. Die Flexibilität des Materials soll durch das Alkyl(meth)acrylat verbessert werden.

Arylhaltige Acrylate werden auch in EP 0 667 966 vorgeschlagen, wobei eine arylhaltige Komponente mit ein oder zwei weiteren Komponenten ohne Arylgruppen kombiniert wird. Auch EP 0 774 983 beschreibt Copolymere, die aus einem arylgruppenhaltigen Acrylmonomer und einem zweiten hydrophilen, insbesondere hydroxygruppenhaltigen Acrylat oder Methacrylat aufgebaut sind. Um Polymere mit einem hohen Brechungsindex bereitzustellen, wird in EP 0 485 197 vorgeschlagen, mindestens zwei Monomere zu copolymerisieren, nämlich ein Arylacrylat und ein Arylmethacrylat. Ein drittes Monomer dient dazu, das Polymer zu vernetzen.

Aus EP 1077952 ist eine Polymerzusammensetzung bekannt, bei der ein spezielles Benzotriazinmonomer in Kombination mit Acrylamiden verwendet wird, um die Eigenschaften zu verbessern.

Allen im Stand der Technik erwähnten Polymeren ist gemeinsam, dass sie aus mindestens drei Monomeren aufgebaut sind, um eine optimale Kombination aus Brechungsindex und mechanischen Eigenschaften zu erzielen.

Keines der bisher bekannten Materialien befriedigt im Hinblick auf eine Kombination aus guten mechanischen Eigenschaften und hohem Brechungsindex.

Aus EP-1 512 704 ist eine Polymerzusammensetzung bekannt, die aus mindestens einem Hauptmonomer
a) mit der Formel 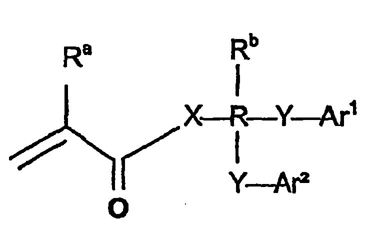 worin X O ist,
   Y jeweils O oder S ist,
   R einen verzweigten Kohlenwasserstoffrest mit 3 Kohlenstoffatomen bedeutet,
   R^{a} Wasserstoff,
   R^{b} Wasserstoff,
   R^{c} Wasserstoff sind und Ar¹, Ar² jeweils unabhängig voneinander eine Phenylgruppe ist, die über eine Bindung an Y gebunden ist,
b) einem vemetzenden Monomer und
c) gegebenenfalls weiteren Monomeren gebildet ist, zur Herstellung eines W-härtenden Materials mit hohem Brechungsindex für optische Filme, Displaysysteme oder prismatische Strukturen.

Es war daher eine Aufgabe der vorliegenden Erfindung, für die Verwendung bei ophthalmologischen Vorrichtungen eine Polymerzusammensetzung bereitzustellen, die exzellente mechanische Eigenschaften mit einem hohen Brechungsindex, bevorzugt über 1,56, vereinigt. Eine weitere Aufgabe war es, für diese Verwendung ein Polymermaterial bereitzustellen, das einerseits so elastisch ist, dass es gefaltet und gerollt werden kann, und andererseits eine solche Festigkeit aufweist, dass es im Auge mechanisch stabil ist. Darüber hinaus war es Aufgabe der Erfindung, für diese Verwendung ein Material bereitzustellen, das biologisch verträglich ist und in einfacher Weise hergestellt werden kann.

Erfindungsgemäß wird für die Verwendung bei ophthalmologischen Vorrichtungen eine Polymerzusammensetzung zur verfügung gestellt, die aus mindestens einem Hauptmonomer a) mit der Formel 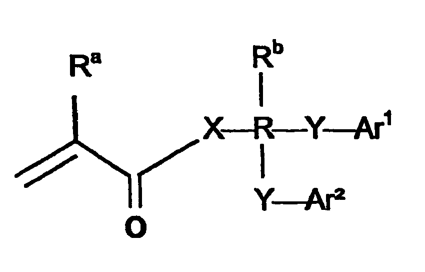 worin X O oder NR^{c} sein kann,
Y Jeweils O, S oder NR^{c} sein kann,
R einen linearen, verzweigten oder cyclischen Kohlenwasserstoffrest mit 1 bis 6 Kohlenstoffatomen bedeutet,
R^{a} Wasserstoff oder einen Methylrest bedeutet,
R^{b} Wasserstoff, C₁-C₅-Alkylrest oder Y-Ar³ bedeuten kann,
R^{c} Wasserstoff, einen linearen, verzweigten oder cyclischen Kohlenwasserstoffrest mit 1 bis 6 Kohlenstoffatomen oder eine Arylgruppe bedeutet,
Ar¹, Ar² und Ar³ jeweils unabhängig voneinander eine Arylgruppe bedeuten, die über eine Bindung oder über (-CH₂)ₙ, wobei n 0, 1, 2 oder 3 sein kann, an Y gebunden ist und wobei die Arylgruppe mit 1 bis 4 Substituenten substituert sein kann, ausgewählt aus C₁-C₅-Alkyl, C₁-C₅-Alkoxy, mono- und disubstituiertem Amino, wobei die Substituenten ausgewählt sein können aus Resten R^{c}, wie oben definiert,
b) einem vernetzenden Monomer und
c) gegebenenfalls weiteren Monomeren gebildet ist, zur Einstellung von Eigenschaften wie Brechungsindex, Oberflächeneigenschaften, Glasübergangstemperatur, Festigkeitseigenschaften, UV-Absorption oder zur Färbung
wobei das Hauptmonomer a) in einer Menge von mindestens 20 Gew.-%, bevorzugt mindestens 40 Gew.-%, besonders bevorzugt mindestens 60 Gew.-% enthalten ist.

Bevorzugt werden als Monomer a) solche Verbindungen eingesetzt, in denen Ar jeweils einen Phenylrest bedeutet, der 0, 1 oder zwei Substituenten aufweist ausgewählt aus Alkyl- und Alkoxyresten.

Besonders bevorzugt werden als Monomere die folgenden Verbindungen eingesetzt

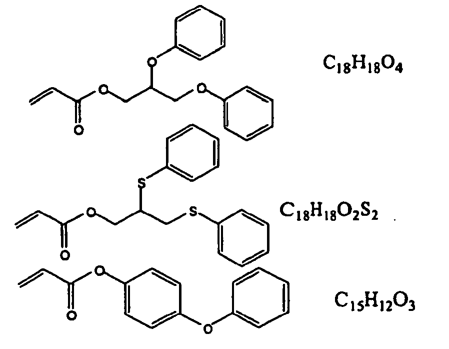

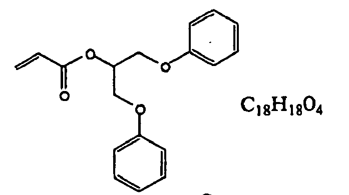

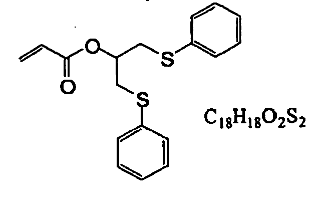

Es wurde überraschenderweise festgestellt, dass das erfindungsgemäße Material besonders gut geeignet ist zur Verwendung im Auge, da es nützliche Eigenschaften kombiniert, die bisher in dieser Kombination nicht zur Verfügung standen. Das erfindungsgemäße Material hat einen sehr hohen Brechungsindex, der es zulässt Augenimplantate mit sehr dünnem Querschnitt herzustellen, die die optischen Anforderungen erfüllen. Weiterhin können mit den erfindungsgemäßen Polymeren Intraokularlinsen hergestellt werden, die überlegene mechanische Eigenschaften haben, sodass die Intraokularlinsen in sehr schonender Weise eingesetzt werden können. Die Polymere eignen sich auch für andere ophthalmische Vorrichtungen, wie Kontaktlinsen, Keratoprothesen, Hornhautringe oder - inlays. Die jeweils optimalen Eigenschaften lassen sich durch Kombination der Monomere gut einstellen.

Das erfindungsgemäße Polymermaterial ist aus den oben genannten Monomeren a), b) und ggf. c) aufgebaut. Ein vernetzendes Monomer - Monomer b) - muss immer verwendet werden, um eine ausreichende Formbeständigkeit zu erzielen. Das Polymer wird im wesentlichen aus der Komponente a) als Hauptmonomer aufgebaut, wobei mindestens 20 Gew.-%, bevorzugt mindestens 40 Gew.-% und besonders bevorzugt mindestens 60 Gew.-% der Monomeren von Monomer a) gebildet werden. Den Rest bilden in der Regel kleinere Anteile weiterer Monomere, die spezielle Eigenschaften beitragen.

In einer bevorzugten Ausführungsform, die als "Homopolymer" bezeichnet wird, besteht das Polymermaterial im wesentlichen aus Monomer a), wobei Monomer b), wie oben ausgeführt, zur Vernetzung mit eingesetzt wird.

Als Homopolymer wird hier ein Polymermaterial bezeichnet, bei dem Monomer a) den wesentlichen Anteil bildet, d.h. mehr als 85%, besonders bevorzugt mehr als 90% der Monomere bildet.

In einer weiteren bevorzugten Ausführungsform wird das Polymer aus Monomerkomponente a), Vernetzer b) und ggf. weiteren Monomeren c) sowie einem weiteren Anteil eines Monomers d) gebildet, das copolymerisiert wird, um besondere Eigenschaften zu erzielen. Das zur Copolymerisierung verwendete zusätzliche Monomer d) ersetzt einen Teil von Monomer a) und muss mit den als Monomer a) und b) verwendeten Verbindungen kompatibel sein. In einer Ausführungsform ist das zusätzlich zugefügte Monomer d) ein Monomer mit einer Struktur wie in Formel I gezeigt, das aber an den Arylresten, zumindest teilweise, mit Halogenen, insbesondere Fluor-, Jod- oder Bromatomen substituiert ist.

Den Rest des Materials bilden Vernetzer und ggf. eine oder mehrere zusätzliche Komponenten ausgewählt aus der Gruppe bestehend aus UV-Licht absorbierenden Verbindungen, Blaulicht absorbierenden Verbindungen, Farbstoffen, Komponenten, die bestimmte Eigenschaften verändern usw.

Es können aber auch andere Monomere, die üblicherweise in Materialien für Augenimplantate Anwendung finden, copolymerisisert werden, Beispiele hierfür finden sich in den genannten Literaturstellen.

Die vorliegende Erfindung stellt somit ein Polymermaterial zur Verfügung, das zur Verwendung als faltbares IOL-Material geeignet ist und in einer Ausführungsform so polymerisiert werden kann, dass im wesentlichen nur zwei Monomere - Monomer a) und Vernetzer b) - zum Einsatz kommen. Damit werden Schwierigkeiten, wie physikalisch/chemische Heterogenität reduziert oder eliminiert.

Die erfindungsgemäße Polymerzusammensetzung kann somit in einer Ausführungsform ein Homopolymer sein, das im wesentlichen nur aus Monomer a) aufgebaut ist und mit Monomer b) vernetzt wird. Die erfindungsgemäße Polymerzusammensetzung kann jedoch auch - neben dem vernetzenden Monomer - aus weiteren Monomeren aufgebaut sein. In jedem Fall ist ein vernetzendes Monomer notwendig, das mit dem Momoner a) und ggf. den weiteren Monomeren copolymerisiert wird.

Als Copolymere werden erfindungsgemäß auch Materialien verstanden, die aus zwei verschiedenen erfindungsgemäßen Monomeren und mindestens einem Vernetzer oder mindestens einem erfindungsgemäßen Monomer, einem Vernetzer und mindestens einem weiteren Monomer polymerisiert wurden.

Die erfindungsgemäßen Polymere können sowohl statistische Polymere als auch Blockcopolymere sein, wobei Blöcke aus erfindungsgemäßen Monomeren und Blöcke aus anderen Monomeren zu vorteilhaften Eigenschaften führen können.

Als vernetzende Monomere werden solche Verbindungen eingesetzt, die mindestens zwei bindungsfähige funktionelle Gruppen aufweisen, Beispiele für geeignete funktionelle Gruppen sind Vinyl-, Acrylat-, Methacrylat-, Hydroxy- und Thiolgruppen. Geeignete vernetzende Verbindungen sind Divinylbenzol, Dithioresorcin, Bisphenol A-Methacrylat. Als vernetzende Monomere können erfindungsgemäß die an sich bekannten Verbindungen eingesetzt werden, unter anderem jede endständig ethylenisch ungesättigte Verbindung mit mehr als einer ungesättigten Gruppe. Geeignete Vernetzungsmittel sind dem Fachmann auf diesem Gebiet bekannt und die üblicherweise verwendeten Monomere können auch für die erfindungsgemäßen Polymere zum Einsatz kommen. Beispiele für bekannte Vernetzer sind z.B. die folgenden bifunktionellen Verbindungen: Ethylenglycoldimethacrylat; Diethylenglycoldimethacrylat; Allylmethacrylat, 1,3-Propandioldimethacrylat; 2,3-Propandioldimethacrylat; 1,6-Hexandioldimethacrylat; 1,4-Butandioldimethacrylat; CH₂=C(CH₃)C(=O)O-(CH₂CH₂O)ₙ-C(=O)C(CH₃)=CH₂, worin n = 1 bis 50 ist und CH₂=C(CH₃)C(=O)O-(CH₂)ₜO-C(=O)C(CH₃)=CH₂, worin t = 3 bis 20 ist und die entsprechenden Acrylate. Geeigneterweise wird der Polymerisationsgrad der Vernetzerverbindung so ausgewählt, dass das zahlenmittlere Molekulargewicht etwa 400, etwa 600 oder, am meisten bevorzugt etwa 1000 ist.

Besonders gute Eigenschaften werden jedoch erhalten, wenn als Vernetzer eine Verbindung der folgenden Formel II 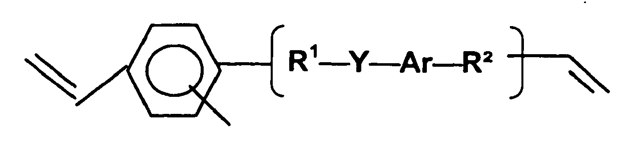 verwendet wird, die an den beiden Enden jeweils eine endständig ungesättigte Gruppe trägt, wobei Y O oder S bedeuten kann, Ar ein aromatischer, insbesondere Phenylrest ist, der mit 0 oder 1 bis 4 Substituenten substituiert sein kann, die ausgewählt sind aus C₁-C₅-Alkylresten, C₁-C₅Alkoxyresten und Halogenen, wobei n eine ganze Zahl von 1 bis 4 sein kann und bevorzugt 1 oder 2 ist. wobei R¹ und R² eine Bindung oder einen (CH₂)ₘ-Rest, worin m 1, 2 oder 3 ist, bedeuten.

Besonders bevorzugt werden als Vernetzer die folgenden Verbindungen verwendet:

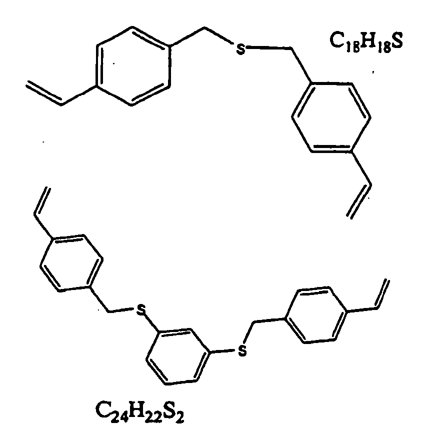

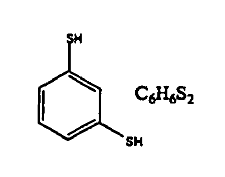

Es wurde gefunden, dass bei Verwendung der erfindungsgemäßen Monomere a) Polymere mit einem Brechungsindex weit über 1,55 entstehen. Der Brechungsindex kann bis zu 1,6 und darüber betragen. In einer besonders bevorzugten Ausführungsform werden Monomere eingesetzt, bei denen Y Schwefel bedeutet. Diese Monomere liefern Polymere mit einem Brechungsindex über 1,6.

In der Regel wird für das erfindungsgemäße Polymer nur eine Art von vernetzendem Monomer eingesetzt. Es ist jedoch auch die Verwendung einer Kombination verschiedener Vernetzer möglich. Z.B. kann eine Mischung aus verschiedenen Monomeren der Formel II oder eine Mischung von Momomeren der Formel II mit einem oder mehreren üblichen Vernetzern verwendet werden.

Allgemein ist die Gesamtmenge der vernetzenden Komponente mindestens 0,1 Gew.-% und abhängig von der Art und Konzentration der verbleibenden Komponenten und den gewünschten physikalischen Eigenschaften kann sie in einem Bereich bis zu 20 Gew.-% liegen. Der bevorzugte Konzentrationsbereich für die vernetzende Komponente ist 0,1 bis 15 Gew.-%. Wenn die Menge an Vernetzer zu gering ist, können die elastischen Eigenschaften des Polymers beeinträchtigt sein. Wenn andererseits der Anteil der vernetzenden Komponente 20 Gew.-% übersteigt, kann das Polymer zu spröde für den vorgesehenen Verwendungszweck sein.

Aus Acrylaten und Methacrylaten aufgebaute hydrophobe Polymere können klebrig sein. Diese Klebrigkeit ist für den Einsatz als Augenimplantat, insbesondere als IOL nachteilig, da beim Aufrollen oder Falten die Oberflächen aneinander kleben und nicht so leicht zu trennen sind. Um die Oberflächeneigenschaften der erfindungsgemäßen Polymerzusammensetzung zu beeinflussen, kann ein weiteres Monomer d) zugefügt werden, das dem Monomer a) ähnlich ist, jedoch mindestens ein Fluoratom oder eine Perflurorgruppe als Substituent trägt. Der Fluorsubstituent kann am Arylrest oder ggf. am Alkylanteil gebunden sein. In einer bevorzugten Ausführungsform wird als fluoriertes Monomer ein solches Monomer eingesetzt, das dem Hauptmonomer strukturell gleicht, das aber als Subsituent Halogen- bzw. Fluoratome aufweist. Eine solche Kombination ist vorteilhaft, da die Monomere miteinander kompatibel sind und zu homogenen Massen führen. Falls halogenierte Monomere verwendet werden, sollte ihr Anteil nicht über 10 Gew.-%, bezogen auf die Menge aller Monomere, liegen. Bevorzugt ist ein Bereich von 3 bis 8%.

Falls das Problem der Klebrigkeit auftritt, kann in einer bevorzugten Ausführungsform entweder ein Anteil des Hauptmonomers a) oder ein Anteil des copolymerisierenden Monomers d) oder ein Anteil der Monomeren a) und der Monomere d) in fluorierter Form copolymerisiert werden.

Weiterhin kann es nützlich sein, einen Anteil eines Monomers gemäß Formel I in jodierter oder bromierter Form zu verwenden. Jodierte und bromierte Verbindungen erhöhen den Brechungsindex des daraus polymerisierten Materials und sind daher vorteilhaft.

Der Anteil der halogenierten Monomere, bevorzugt fluorierten Monomere, liegt, falls vorhanden, in einem Bereich von 0,05 bis 10 Gew.-%, bezogen auf das Gewicht der gesamten Polymerzusammensetzung. Bevorzugt sind halogenierte Monomere in einem Anteil von 0,1 bis 3% enthalten. Wenn der Anteil des halogenierten Monomers zu hoch ist, wird der Brechungsindex zu stark beeinflusst. Andererseits ist bei einer zu geringen Einsatzmenge der Einfluss auf die Oberflächeneigenschaften zu gering, um spürbar zu sein. Abhängig von den verwendeten Hauptmonomeren können Art und Menge des halogenierten Monomers vom Fachmann in wenigen Routineversuchen eingestellt werden.

Als weitere Inhaltsstoffe für das erfindungsgemäße Polymermaterial können die für diese Art von Polymersystemen bekannten Inhaltsstoffe, wie Initiatoren, Farbstoffe etc. genannt werden. Generell werden alle Zusatzstoffe in die Polymere einpolymerisiert und nicht getrennt zugesetzt, damit Verbindungen nicht ausgelaugt werden können.

Die Polymerisation wird üblicherweise durch einen Initiator gestartet, der der zu polymerisierenden Masse zugesetzt wird. Für die erfindungsgemäße Zusammensetzung können sowohl Verbindungen, die durch Wärme aktivierbar sind, als auch Verbindungen, die durch Licht aktivierbar sind, eingesetzt werden. Da im Bereich des Auges verwendete Polymere in der Regel UV-Absorber enthalten, ist es ungünstig, UV-Initiatoren zu verwenden. Bevorzugt werden daher entweder dem Fachmann an sich bekannte Blaulichtinitiatoren oder mit IR-Strahlen oder Wärme aktivierbare Verbindungen verwendet.

Bevorzugte thermische Initiatoren sind z.B. Verbindungen mit Peroxyrest, wie t-Butyl(peroxy-2-ethyl)hexanoat und Di(tert.-butylcyclohexyl)peroxydicarbonat, die häufig zur Polymerisation von Intraokularlinsen verwendet werden. Als Fotoinitiator sind die üblicherweise verwendeten geeignet, wie Azoverbindungen, z.B. MAIB, Phosphinoxidverbindungen wie Benzoylphosphinoxid, insbesondere 2,4,6-Trimethylbenzoyldiphenylphosphinoxid. Initiatoren werden in an sich bekannten Mengen, z.B. in einer typischen Ausführungsform in einer Menge von 5 Gew.-% oder weniger zugegeben.

UV-Absorber werden häufig für Polymere, die im Auge zum Einsatz kommen, zugegeben, um das Auge vor Schäden durch UV-Strahlung zu schützen. Häufig werden dazu Benzotriazolverbindungen ausgewählt. Ein bekannter reaktiver UV-Absorber ist z.B. 2-(2'-Hydroxy-3'-methallyl-5'-methylphenyl)benzotriazol. UV-Absorber sind typischerweise in einer Menge von 0,1 bis 5 Gew.-% vorhanden.

Die Art und Menge der oben erwähnten gegebenenfalls zusätzlichen Komponenten werden durch die gewünschten Eigenschaften des fertigen ophthalmischen Implantats bestimmt. Bevorzugt werden Inhaltsstoffe und deren Anteile so ausgewählt, dass die Polymere der vorliegenden Erfindung die gewünschten optischen und mechanischen Eigenschaften besitzen, die die Materialien der vorliegenden Erfindung besonders geeignet zur Verwendung im Auge machen.

Das Linsenmaterial hat bevorzugt einen Brechungsindex in trockenem Zustand von mindestens 1,60. Wenn ein höherer Anteil schwefelhaltiger Monomere zu dem erfindungsgemäßen Material polymerisiert wird, können auch Werte von mehr als 1,60 erreicht werden, was besonders bevorzugt ist.

Für einen gegebenen optischen Durchmesser sind Optiken, die aus Materialien mit einem Brechungsindex von unter 1,50 hergestellt werden, notwendigerweise dicker als Optiken der gleichen Brechkraft, die aus Materialien mit einem höheren Brechungsindex hergestellt werden. Je dünner die Optik sein kann, desto kleiner wird der Einschnitt sein, über den das Implantat in das Auge gebracht wird.

Das erfindungsgemäß zur Verfügung gestellte Polymer hat durch Verwendung der Monomere a) und b) vorteilhafte mechanische Eigenschaften. Insbesondere ist das Polymer so ausgebildet, dass ein daraus hergestelltes Augenimplantat im Allgemeinen nicht bricht, reißt oder splittert, wenn es gefaltet oder gerollt wird.

Intraokularlinsen, die aus den Materialien der vorliegenden Erfindung konstruiert werden, können in an sich bekannter Weise aufgebaut sein, wobei der Aufbau davon abhängt, ob sie gerollt oder zu einem kleinen Querschnitt gefaltet werden, der durch einen relativ kleineren Einschnitt passen kann. Die Intraokularlinsen können z.B. einen einstückigen oder mehrstückigen Aufbau haben und weisen Optik- und Haptikkomponenten auf. Die Optik ist der Teil, der als Linse dient. Die Haptiken sind an der Optik befestigt und halten die Optik an ihrem richtigen Platz im Auge. Optik und Haptik(en) können aus dem gleichen Polymer gebildet sein oder aus verschiedenen Materialien bestehen. Bei einer als mehrstückig bezeichneten IOL werden Optik und Haptik(en) getrennt hergestellt und anschließend die Haptiken an der Optik befestigt. Bei einer einstückigen Linse werden Optik und Haptiken aus Polymer gebildet. Die Formung und Bearbeitung von Optik und Haptik erfolgt in dem Fachmann wohlbekannter Art und Weise.

Mit den erfindungsgemäßen Monomeren können sowohl hydrophobe als auch hydrophile Polymerzusammensetzungen hergestellt werden. Falls ein hydrophobes Polymer gewünscht ist, kann Monomer a) als Homopolymer oder mit weiteren hydrophoben Comonomeren verarbeitet werden. Falls ein hydrophiles Polymermaterial gewünscht ist, wird das erfindungsgemäße Monomer a) mit einem hydrophilen Monomer copolymerisiert, das z.B. ein Acrylat oder Methacrylat mit Hydroxygruppen sein kann.

Ein weiterer für die Verarbeitungseigenschaften von Polymeren wichtiger Parameter ist die Glasübergangstemperatur. Die Glasübergangstemperatur beeinflusst die Flexibilität des Materials, Wenn die Glasübergangstemperatur sehr hoch ist, ist das Material bei Körpertemperatur und Raumtemperatur spröde, wenn die Glasübergangstemperatur sehr niedrig ist, lässt sich das Material bei üblichen Temperaturen kaum verarbeiten. Zur Verwendung als Augenimplantate sind daher Polymere wünschenswert, die eine T_{g} von 15°C oder weniger, bevorzugt 10°C oder weniger haben, da in diesem Fall Polymere erhalten werden, die gut bearbeitet werden können und trotzdem noch ihre elastischen Eigenschaften bei Körpertemperatur aufrechterhalten. Um bei dem aus den oben genannten Monomeren hergestellten Polymer eine optimale Glasübergangstemperatur einzustellen, wird bevorzugt ein Monomer zugefügt, das die Glasübergangstemperatur in den gewünschten Bereich bringt. Hierzu geeignete Monomere sind dem Fachmann an sich bekannt und das geeignete Monomer und dessen Einsatzmenge kann in wenigen Routineversuchen aufgefunden werden.

Erfindungsgemäße Augenimplantate können noch weiter verbessert werden, indem den Polymeren Nanopigmente, wie sie an sich bekannt sind, zugesetzt werden. Aus DE 101 29 787 ist es bekannt, optische Bauelemente in Materialien für Augenimplantate einzuarbeiten. Als optische Bauelemente werden im wesentlichen lichtdurchlässige Füllstoffe mit einer höheren Brechzahl als der des umgebenden Materials und mit einer Partikelgröße, bei welcher im wesentlichen keine Lichtstreuung im Bauelementmaterial stattfindet, verwendet. Der optisch klare bzw. lichtdurchlässige Füllstoff besitzt eine hohe Elektronendichte, weiche zu einer erhöhten Brechzahl führt. Diese hohe Elektronendichte kann durch schwer lösliche Oxide mit hochgeladenen Kationen, beispielsweise durch Schwermetall-, insbesondere Blei- und Wismutverbindungen erreicht werden. Diese Schwermetallverbindungen liegen in kristalliner, insbesondere nanokristallin abgeschiedener Form, beispielsweise als Silicate, Germanate, Aluminate oder Titanate vor. Die Schwermetalle sind in der Kristallmatrix fest integriert und werden im biologischen Milieu des Auges nicht herausgelöst. Die Füllstoffe beeinträchtigen daher nicht die biologische Verträglichkeit des lichtdurchlässigen Bauelementematerials bzw. Implantatmaterials, in welchem sie in feinverteilter Partikelform insbesondere als Nanopartikel verteilt sind. Ein vorzugsweise zur Anwendung kommender Füllstoff ist Rutil. Dieser Füllstoff ist biokompatibel. Er ist inert und schwer löslich, thermisch stabil und somit autoklavierbar. Ferner ist er in größeren Mengen verfügbar. Dieser Füllstoff ist nanokristallin abscheidbar und somit technisch in einer Partikelgröße herstellbar, bei welcher praktisch keine Lichtstreuung im Bauelementematerial verursacht wird.

Bei Verwendung von 20 Vol.-% Rutil als Füllstoff in einem Acrylat mit einer Brechzahl von n = 1,5 lässt sich die Brechzahl des Acrylats durch den Füllstoff auf 1,78 erhöhen. Dadurch ist es möglich, den wirksamen Brechzahlunterschied zwischen dem Augenimplantat und dem umgebenden Kammerwasser um den Faktor 2 bis 3,5 zu erhöhen. Dies lässt es zu, beispielsweise Intraokularlinsen mit verringerter Dicke und verbesserter Faltmöglichkeit herzustellen.

In einer bevorzugten Ausführungsform werden daher dem Polymer, das zu einer IOL verarbeitet werden soll, bis zu 20 Gew.-%, bevorzugt 5 bis 15 Gew.-% Nanopartikel, wie oben beschrieben, zugefügt.

Ein weiterer Gegenstand der Erfindung ist ein Verfahren zur Herstellung der erfindungsgemäßen Polymerzusammensetzung. Verfahren zur Herstellung von Acrylat- und Methacrylatpolymeren sind an sich bekannt. Für die Herstellung von Augenimplantaten werden insbesondere Bulkpolymerisation und Emulsionspolymerisation, bevorzugt Emulsionspolymerisation in Betracht gezogen.

In einer bevorzugten Ausführungsform werden Monomere unter Verwendung eines Initiators I zu Präpolymeren umgesetzt und soweit möglich von ihrem Restmonomergehalt befreit. Anschließend werden die Präpolymere, gegebenenfalls unter Zusatz von Monomeren, die bestimmte Eigenschaften beeinflussen, zum Endpolymer in Gegenwart eines Initiators II umgesetzt. Die Initiatoren I und II können gleich oder verschieden sein, abhängig von der Art der eingesetzten Monomere und den gewünschten Eigenschaften des Polymers. Bevorzugt sind Initiator I und Initiator II gleich. Diese Art des Verfahrens führt zu einem sehr geringen Restmonomergehalt, was für die geplante Verwendung vorteilhaft ist.

Weiterhin entstehen durch Verwendung eines zweistufigen Verfahrens, bei dem in einer ersten Stufe Präpolymere hergestellt und diese dann mit monomeren Vernetzern weiter polymerisiert werden, homogene Materialien. Für den Einsatz als Augenimplantat ist die Homogenität des Materials ein wichtiger Parameter. Inhomogene Bereiche in einem Polymer führen zu optischen Fehlern, was für Augenimplantate nicht akzeptabel ist.

Erfindungsgemäß werden Polymerzusammensetzungen bereitgestellt, die gute mechanische Eigenschaften mit hoher Brechkraft vereinigen. Diese Polymere können in einfacher Weise hergestellt werden, unter Verwendung üblicher Verfahren.

Die erfindungsgemäßen Polymerzusammensetzungen sind besonders gut geeignet zur Verwendung als Hornhauttransplantate und Intraokularlinsen. Gegenstand der Erfindung ist daher auch die Verwendung einer Polymerzusammensetzung, wie oben definiert, als Hornhautimplantat oder IOL.

Die Erfindung wird weiter durch die folgenden Beispiele erläutert, die jedoch in keiner Weise als beschränkend interpretiert werden sollen.

Die erhaltenen Produkte wurden mit ¹H- und ¹³C-NMR-Spektroskopie untersucht. Es wurde ein Spektrometer INNOVA 500 (Varian Inc.) verwendet und die Messungen wurden an diesem Spektrometer bei Raumtemperatur (21 °C) mit folgenden Messfrequenzen durchgeführt: ¹H-NMR: 499,84 MHz, ¹³C-NMR: 125,69 MHz. Als Lösungsmittel wurden CD₂CL₂ und CD₃OD verwendet.

### Beispiel 1

### Synthese von

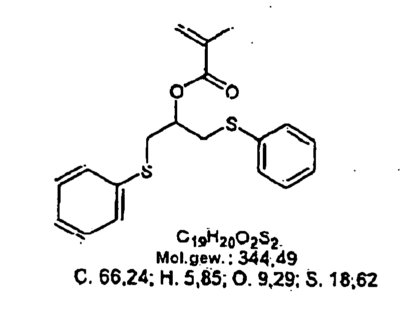

### Reaktionsgleichung

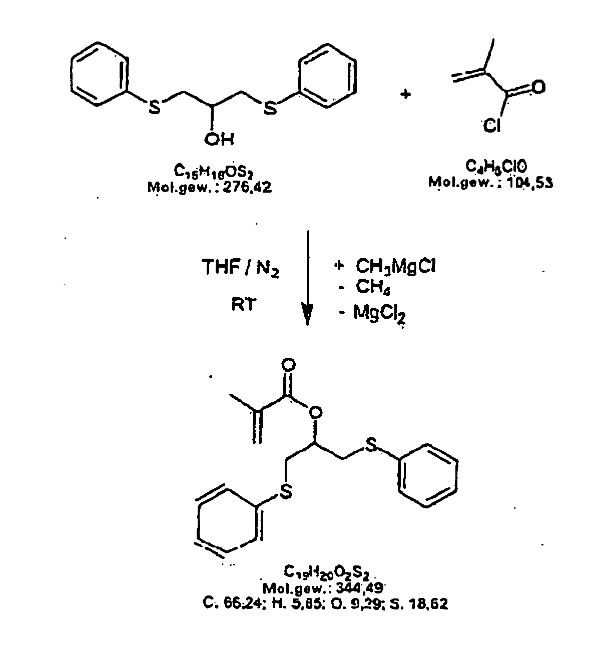

### Reagenzien:

### Methacryloylchlorid

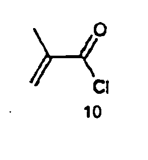

### Methylmagnesiumchlorid

### Experimenteller Teil

### (2-Methacrylsäure-2-phenylsulfanyl-1-phenylsulfanylmethyl-ethylester)

1,3-Bisphenylsulfanyl-propan-2-ol wurde mit Methylmagnesiumchlorid und Methacrylsäurechlorid in äquimolaren Anteilen umgesetzt (Ansatz je 0,03618 Mol).

1,3-Bisphenylsulfanyl-propan-2-ol (MW = 276,42): (10 g = 0,03618 Mol) wurden in THF, das vorher über Na/K destilliert worden war, gelöst. Zu dieser Lösung wurden 0,03618 Mol Methylmagnesiumchlorid [Acros, 22 gew.-%ige Lösung in THF] (MW = 74,79) = (2,71 g (entspricht 12,32 g einer 22 gew.-%igen Lösung)) zugegeben. Diese Lösung wurde dann durch einen Tropftrichter zu folgender Lösung langsam zugegeben: 0,03618 Mol Methacryloylchlorid [97,0% (GC)] (MW = 104,53) = (3,78 g (d.h. 3,89 g des 97,0%igen Präparats)) in ca. 100 ml THF gelöst. Die Reaktionslösung erwärmte sich nicht nennenswert, eine Kühlung war nicht nötig; die Lösung blieb klar, leicht gelblich. Bei Raumtemperatur wurde unter Stickstoffatmosphäre gerührt. Die Reaktion wurde über DC kontrolliert. Anschließend wurde ca. 2 h lang auf 45 bis 50°C erwärmt. Es wurde weiter über Nacht bei Raumtemperatur unter N₂-Atmosphäre gerührt. Die Filtration der Reaktionslösung erfolgte über eine mit Aluminiumoxid [Acros, Aluminiumoxid, activated, basic] und Seesand beschichtete G3-Fritte, die vorher mit THF aufgeschlämmt wurde. Anschließend wurde das THF am Rotationsverdampfer entfernt und das Produkt dann mit Säulenchromatographie weiter gereinigt.

### Beispiel 2

### Synthese von

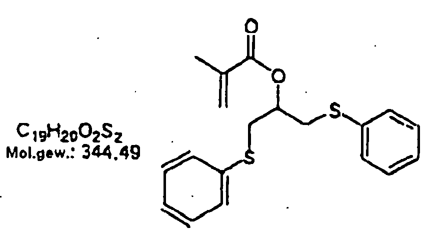

### Reaktionsgleichung

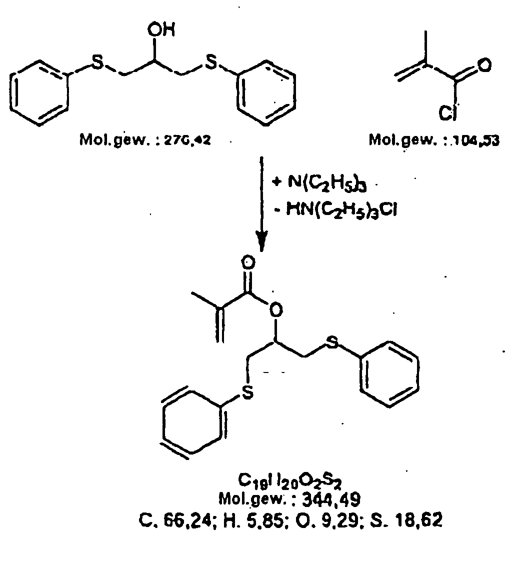

### Chemikalien

### Methacryloylchlorid (wie oben definiert)

### Experimenteller Teil

1,3-Bisphenylsulfanyl-propan-2-ol wurde mit Methacryloylchlorid umgesetzt. In einen vorher ausgeheizten 250-ml-Dreihalskolben mit Kühler und N₂-Einleitung wurden die folgenden Reagenzien gegeben: 0,03 Mol Methacryloylchlorid (3,88 g, 97%ig), ca. 60 ml inhibitorfreies vorher destilliertes THF als Lösemittel. In einen Tropftrichter wurden 8,29 g 1,3-Bisphenylsulfanyl-propan-2-ol (0,030 Mol) und 2,94 g Triethylamin (0,030 Mol) (99 gew.-%ig) zugefügt. Die Lösung aus dem Tropftrichter wurde langsam in den Dreihalskolben zutropfen gelassen. Da die Reaktion nicht exotherm war, war keine Kühlung notwendig. Es bildete sich ein weißer Niederschlag aus (NEt₃)HCl. Bei Raumtemperatur wurde 1,5 Stunden lang weiter gerührt und der entstehende Niederschlag, ca. 2,8 g (NEt₃)HCl dann abfiltriert und anschließend das THF am Rotationsverdampfer entfernt. Man erhielt eine gelbe zähe Flüssigkeit. Die Rohausbeute war 14 g. Zur Reinigung des erhaltenen Produktes wurde die erhaltene Flüssigkeit (14 g) in ca. 50 ml CH₂Cl₂ gelöst und dann mit 5%iger NaHCO₃-Lösung ausgeschüttelt. Es bildete sich eine Emulsion, die durch Zugabe von NaCl gebrochen wurde. Anschließend wurde über Na₂SO₄ getrocknet und dann am Rotationsverdampfer und anschließend mit einer Hybridölpumpe eingeengt. Man erhielt eine gelbe viskose Flüssigkeit in einer Ausbeute von ca. 9 g, was 0,0267 Mol oder 89% der Theorie entsprach. Das Produkt wurde anschließend noch chromatographisch aufgereinigt.
¹H-NMR für C₁₉H₂₀O₂S₂ (Molekulargewicht 344,49) in CD₂Cl₂
¹H-NMR: 499,84 MHz in CD₂Cl₂
7,36-7,34 ppm (4 H, m, H3), 7,27-7,24 ppm (4 H, m, H2), 7,19-7,17 ppm (2 H, m, H1), 5,90 ppm (1 H, m, H9), 5,48 ppm (1 H, m, H9'), 5,12 ppm (1 H, m, H6), 3,29 ppm (4 H, m, H5), 1,80 ppm (3 H, m, H8)
¹³C-NMR: 125,69 MHz in CD₂Cl₂
166,66 ppm (C7), 136,22 ppm (C4), 135,73 ppm (C8), 129,89 ppm (C3), 129,28 ppm (C2), 126,70 ppm (C1), 126,09 ppm (C9), 72,17 ppm (C6), 36,53 ppm (C5), 18,16 ppm (C10)

### Beispiel 3

### Synthese von

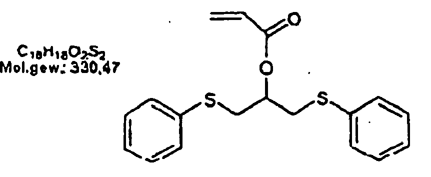

### Reaktionsgleichung

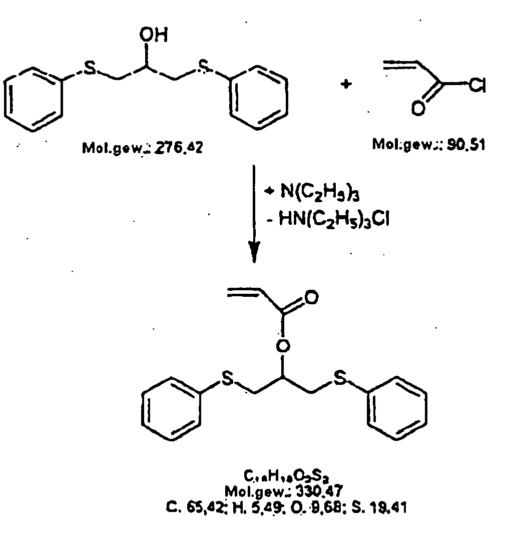

### Reagenzien

### Acryloylchlorid

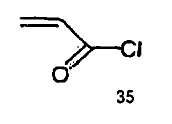

### Experimenteller Teil

In einen vorher ausgeheizten Dreihalskolben mit Kühler und N₂-Einleitung wurden 0,03 Mol Acryloylchlorid, wie vorher definiert, (2,828 g, 96 gew.-%ig) und 50 bis 100 ml inhibitorfreies THF (vorher dest.) als Lösungsmittel gegeben. In einen Tropftrichter wurden 8,29 g 1,3-Bisphenylsulfanylpropan-2-ol = 0,030 Mol und 0,030 Mol Triethylamin [99%], (4,1 ml) gegeben. Aus dem Tropftrichter wurde die Lösung langsam zutropfen gelassen. Da die Reaktion nicht exotherm ist, ist keine Kühlung notwendig. Es bildete sich ein weißer Niederschlag aus (NEt₃)HCl. Es wurde weitere 1,5 h bei Raumtemperatur gerührt und anschließend der Niederschlag abfiltriert und das THF am Rotationsverdampfer entfernt. Man erhielt eine gelbe zähe Flüssigkeit in einer Ausbeute von ca. 9,5 g. Zur Reinigung wurde das Produkt in 50 ml inhibitorfreiem THF gelöst und über eine Alox-Fritte filtriert, um mögliche Inhibitorreste zu entfernen. Das THF wurde am Rotationsdampfer entfernt und anschließend mit einer Hybridpumpe (1,5 bis 1,5 mbar) restliches THF entfernt. Man erhielt eine gelbe viskose Flüssigkeit in einer Ausbeute von ca. 6,8 g, was 0,02067 Mol oder 68,9% der Theorie entspricht.
¹HNMR für C₁₉H₂₀O₂S₂ (Molekulargewicht 344,49) in CD₂Cl₂ 7,33-7,37 ppm (4 H, m, H3), 7,24-7,29 ppm (4 H, m, H2), 7,16-7,21 ppm (2 H, m, H1)
6,225 ppm (1 H, dd, ²J(7',6) = 17,1 Hz, ²J(7', 7) = 1,22 Hz, H7' (trans))
5,93 ppm (1 H, dd, ²J(6,7) = 10,5 Hz, ²J(6,7') = 17,3 Hz, H6)
5,755 ppm (1 H, dd, ²J(7,6) = 10,4 Hz, ²J(7,7') = 1,22 Hz, H7(cis))
5,13-5,15 ppm (1 H, m, H5)
3,27 ppm (4 H, m, H4)

### Beispiel 4

### Synthese von

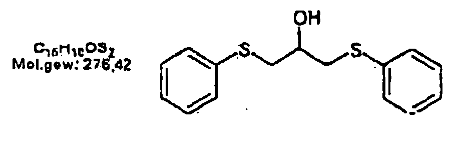

### Reaktionsgleichung

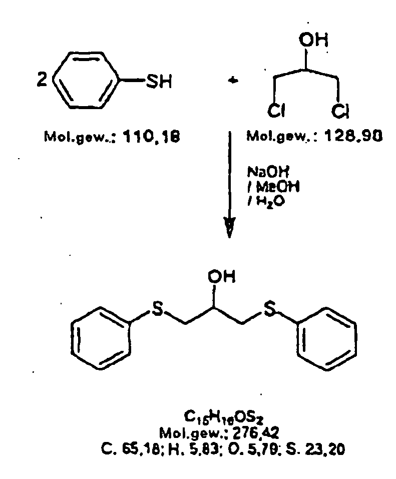

### Reagenzien

### Thiophenol [>98%]

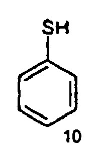

### 1,3-Dichlor-2-propanol [99%]

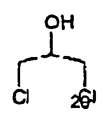

### Natriumhydroxid, Pellets mit 99,998%

### Experimenteller Teil

In einen 500-ml-Dreihalskolben mit Kühler, Tropftrichter und N₂-Einleitung wurden unter N₂-Atmosphäre zuerst gegeben: 0,05 Mol 1,3-Dichlor-2-propanol [99%] (6,449 g) gelöst in 100 ml Methanol. In einen Tropftrichter wurden gegeben: 0,1 Mol Thiophenol [98%], (11,018 g) in 50 ml destilliertem Wasser gelöst, zusammen mit 0,1 Mol NaOH; die Mischung wurde langsam der 1,3-Dichlor-2-propanol-Lösung zugetropft. Nach dem 2/3 der Mischung zugegeben worden waren, entstand ein weißer Niederschlag mit leicht rosa Verfärbung und die Reaktionslösung erwärmte sich. Weitere 50 ml Methanol wurden zugegeben und es wurde über Nacht am Rückfluss (70°C) erhitzt. Der Verlauf der Reaktion wurde über DC verfolgt, wobei CH₂Cl₂ als Laufmittel und Thiophenol als Vergleich verwendet wurden. Am nächsten Tag war die Reaktionslösung klar, leicht gräulich und mit öligen Perlen versetzt. Die Reaktionszeit betrug ca. 20 Stunden bei 70°C.

Die Lösung wurde zuerst am Rotationsverdampfer eingeengt und dann mit CH₂Cl₂ ausgeschüttelt (dreimal mit ca. 100 ml) und über Na₂SO₄ getrocknet.

Dann wurde zuerst am Rotationsverdampfer und dann an der Hybridölpumpe (p = 1,8-1,6 mbar, T = 30°C) eingeengt.

### Charakterisierung:

1,3-Bisphenylsulfanyl-propan-2-ol: 13,63 g einer gelblichen öligen Flüssigkeit (0,0481 Mol); Ausbeute 98,6% der Theorie bezogen auf 0,05 Mol 1,3-Dichlor-2-propanol;
¹H-NMR: 7,32-7,35 ppm (4 H, m, H3), 7,24-7,29 ppm (4 H, m, H2), 7,17-7,21 ppm (2 H, m, H1), 3,78-3,82 ppm (1 H, m, ²J(5,6) = 3,7 Hz, H6), 3,18-3,22 ppm (2H, dd, ²J(4,4') = 13,9 Hz, ²J(4,5) = 4,9 Hz, H5), 3,01-3,05 ppm (2 H, dd, ²J(4',4) = 13,7 Hz, ²J(4',5) = 7,3 Hz, H5'), 2,71 ppm (1 H, d, ²J(6,5) = 3,4 Hz, -OH)
¹³C-NMR: 125,69 MHz in CD₃OD
137,51 ppm (C4), 130,50 ppm (C3), 130,02 ppm (C2), 127,17 ppm (C1), 70,08 ppm (C6), 40,45 ppm (C5)

### Beispiel 5

### Synthese von

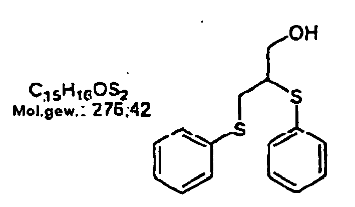

### Reaktionsgleichung

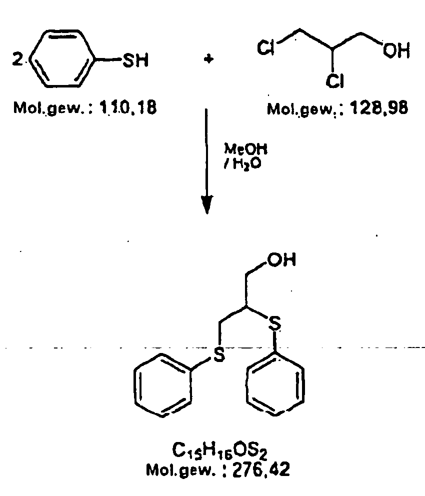

### Reagenzien

### Thiophenol (wie in Beispiel 4)

### 2,3-Dichlor-1-propanol [97,0% (GC)]

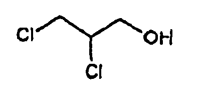

### Natriumhydroxid, Pellets 99.998%

### Experimenteller Teil

In einen 500-ml-Dreihalskolben, der mit Kühler, Tropftrichter, N₂-Einleitung ausgestattet war, wurden unter N₂-Atmosphäre 0,05 Mol 2,3-Dichlor-1-propanol [97% (GC) (6,449 g)] gelöst in 100 ml Methanol gegeben. In einen Tropftrichter wurden 0,1 Mol Thiophenol [98%] (11,018 g) in 50 ml destilliertem Wasser gelöst und 0,1 Mol NaOH, (4,0 g) gelöst in 50 ml destilliertem Wasser, zugegeben. Diese Lösung wurde langsam der 1,3-Dichlor-2-propanol-Lösung zugetropft. Dabei entstand eine leicht rosa Verfärbung und die Reaktionslösung erwärmte sich. Der Verlauf der Reaktion wurde über DC verfolgt, wobei CH₂Cl₂ als Laufmittel und Thiophenol als Vergleich verwendet wurden. Weitere 50 ml Methanol wurden zugegeben und es wurde über Nacht am Rückfluss (70°C) erhitzt. Am nächsten Tag war die Reaktionslösung klar, leicht gräulich und mit öligen Perlen versetzt. Die Reaktionszeit war bei 70°C ca. 20 h.

Die Lösung wurde zuerst am Rotationsverdampfer eingeengt und dann mit CH₂Cl₂ ausgeschüttelt (dreimal mit ca. 100 ml) und über Na₂SO₄ getrocknet. Dann wurde zuerst an einem Rotationsverdampfer und dann an der Hybridölpumpe eingeengt.

Es wurden 12,85 g (0,0465 Mol) einer gelblichen öligen Flüssigkeit erhalten. Dies entsprach einer Ausbeute von 93% der Theorie bezogen auf 0,05 Mol 2,3-Dichlor-1-propanol.

### Charakterisierung:

Das Produkt wurde mit ¹H-NMR + ¹³C-NMR in CD₃-OD untersucht: Es lag ein Isomerengemisch vor mit ca. 80% der Verbindung zu 20% des 2-Propanolderivats.

Der Brechungsindex wurde bestimmt mit: η = 1,6255/T = 25,3°C

### Beispiel 6

### Synthese von

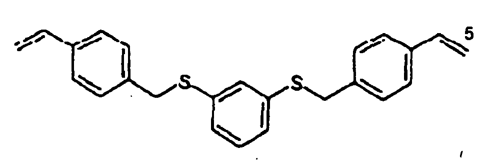

### Reaktionsgleichung

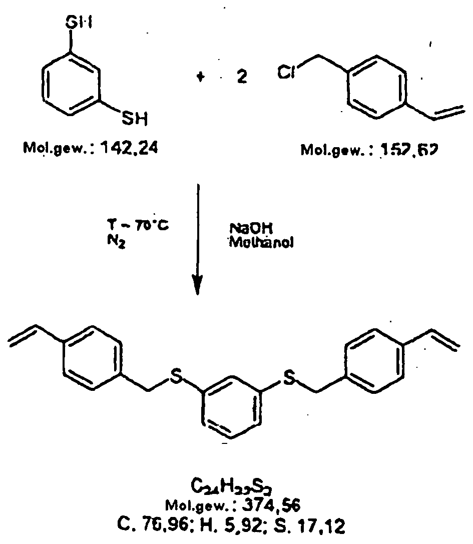

### Reagenzien

### 1,3-Benzoldithiol, 99%

### 4-Vinylbenzylchlorid, technisch 90% (GC)

### Experimenteller Teil

In einen 250-ml-Dreihalskolben, der mit Kühler, Tropftrichter und N₂-Einleitung ausgestattet war, wurden unter Rühren und N₂-Atmosphäre nacheinander 0,01 Mol 1,3-Dithiobenzol [99%] (1,437 g) und 0,02 Mol NaOH (ca. 0,8 g) gelöst in ca. 30 ml destilliertem Wasser gegeben.

Es entstand ein farbloser kristalliner Niederschlag. Der Ansatz wurde auf 40°C erwärmt und 80 ml Methanol wurden zugegeben. Dabei entstand eine milchige Lösung. Anschließend wurden 0,02 Mol 4-Vinylbenzylchlorid [≥90%] (3,0524 g) zugegeben, wobei sich eine Emulsion bildete. Um den restlichen Anteil des 4-Vinylbenzylchlorids zu lösen, wurden weitere 60 ml Methanol zugegeben. Der Ansatz wurde auf 70°C erwärmt und dann über Nacht am Rückfluss gehalten (ca. 65°C). Nach 23 h Reaktionszeit wurde die Reaktionslösung abfiltriert und mit Ether (dreimal mit je 80 ml) extrahiert. Die vereinigten Etherphasen wurden dann zuerst mit 80 ml 1 n NaOH und dann mit destilliertem Wasser ausgeschüttelt und dann über Natriumsulfat getrocknet und am Rotationsverdampfer eingeengt (35°C, p = 700-750 mbar).

Es wurden 1,82 g einer gelblichen öligen Flüssigkeit erhalten. Methanol wurde zugegeben und es wurde auf 66°C erhitzt. Dabei löste sich fast alles und die gelbe Farbe verschwand. Anschließend wurde heiß filtriert und der Filter leicht erwärmt. Dabei fielen sofort farblose Nadeln aus. Es wurde über eine G3-Fritte abgesaugt und dann an einer Hybridölpumpe getrocknet (erst p = 120 mbar, dann 0,12 mbar). Die Ausbeute war 0,76 g (0,00203 Mol), was 20,3% der Theorie entsprach.

¹H-NMR: 499,84 MHz in CD₂Cl₂

7,33 ppm (4H, d, ²J(4,5) = 8,1 Hz, H4), 7,22 ppm (4 H, d, ²J(5,4) = 8,1 Hz, H5), 7,185-7,20 ppm (1 H, m, H11), 7,01-7,16 ppm (3 H, m, 2 H9, 1 H10), 6,68 ppm (2 H, q, ²J(2,1') = 11,0 Hz, ²J(2,1) = 17,6 Hz, H2), 5,73 ppm (2 H, dd, ²J(1,1') = 1,0 Hz, ²J(1,2) = 17,6 Hz, H1), 5,22 ppm (2 H, dd, ²J(1',2) = 11,0 Hz, ²J(1',1) = 1,0 Hz, H1'), 4,05 ppm (4 H, s, H7),

¹³C-NMR: 125,69 MHz in CD₂Cl₂

C3 137,42 ppm, C6 137,25 ppm, C8 136,87 ppm, C2 136,58 ppm, C11 130,23 ppm, C10 129,33 ppm, C5 129,26 ppm, C9 127,59 ppm, C4 126,53 ppm, C1 113,93 ppm, C7 38,56 ppm

### Beispiel 7

1,0 g des Monomers von Beispiel 1 wurden mit 0,2% Irgarcure 2022 gemischt und zwischen zwei silanisierte Objektträger, die durch einen Spacer mit 0,9 mm Dicke getrennt waren, gefüllt. Die Photopolymerisation wurde unter N₂-Atmosphäre mit einer UV-Lampe der Marke "Super Actinic" (Lampe TL-D15W/03, λₘₐₓ = 420 nm, Bestrahlungsabstand 18 cm) 2 Stunden lang durchgeführt. Die Vernetzung der Polymere wurde durch Zugabe von 3% Ethylenglycoldimethacrylat erreicht.

## Patentansprüche

1. Verwendung einer Polymerzusammensetzung erhalten durch Polymerisation mindestens eines Hauptmonomers a) mit der Formel 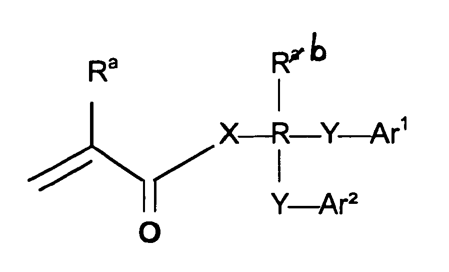 worin X O oder NR^{c} sein kann,
Y jeweils O, S oder NR^{c} sein kann,
R einen linearen, verzweigten oder cyclischen Kohlenwasserstoffrest mit 1 bis 6 Kohlenstoffatomen bedeutet,
R^{a} Wasserstoff oder einen Methylrest bedeutet,
R^{b} Wasserstoff, C₁-C₅-Alkylrest oder Y-Ar³ bedeuten kann,
R^{c} Wasserstoff, einen linearen, verzweigten oder cyclischen Kohlenwasserstoffrest mit 1 bis 6 Kohlenstoffatomen oder eine Arylgruppe bedeutet,
Ar¹, Ar² und Ar³ jeweils unabhängig voneinander eine Arylgruppe bedeuten, die über eine Bindung oder über (-CH₂)ₙ, wobei n 0, 1, 2 oder 3 sein kann an Y gebunden ist und wobei die Arylgruppe mit 1 bis 4 Substituenten substituert sein kann, ausgewählt aus C₁-C₅-Alkyl, C₁-C₅-Alkoxy, mono- und disubstituiertem Amino, wobei die Substituenten ausgewählt sein können aus Resten R^{c}, wie oben definiert,
b) einem vernetzenden Monomer und
c) gegebenenfalls weiteren Monomeren zur Einstellung von Eigenschaften wie Brechungsindex, Oberflächeneigenschaften, Glasübergangstemperatur, Festigkeitseigenschaften, UV-Absorption, und/oder zur Färbung
für ophthalmologische Vorrichtungen.

2. Verwendung einer Polymerzusammensetzung nach Anspruch 1, wobei in Monomer a) Y S bedeutet.

3. Verwendung einer Polymerzusammensetzung nach Anspruch 1 oder Anspruch 2, wobei als Monomer a) eine der folgenden Verbindungen 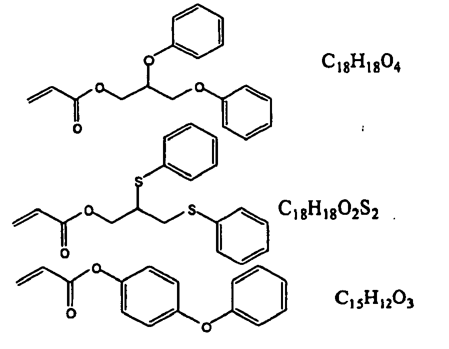 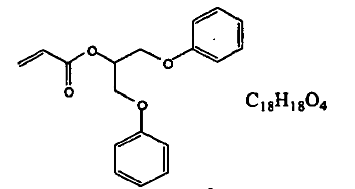 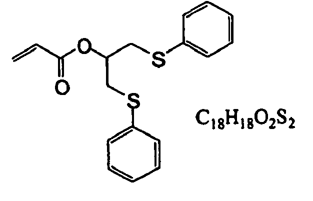 oder eine Mischung davon verwendet wird.

4. Verwendung nach einem der vorhergehenden Ansprüche, wobei als Monomer c) POEMA oder HEMA eingesetzt wird.

5. Polymerzusammensetzung nach einem der vorhergehenden Ansprüche, **dadurch gekennzeichnet, dass** als vernetzendes Monomer eine Verbindung der folgenden Formel II 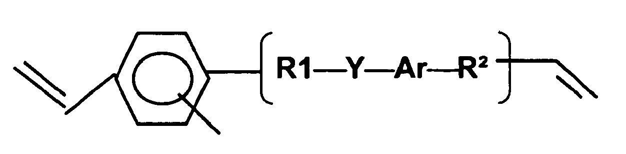 verwendet wird, die an den beiden Enden jeweils eine endständig ungesättigte Gruppe trägt, wobei Y O oder S bedeuten kann, Ar ein aromatischer, insbesondere Phenylrest ist, der mit 0 oder 1 bis 4 Substituenten substituiert sein kann, die ausgewählt sind aus C₁-C₅-Alkylresten, C₁-C₅-Alkoxyresten und Halogenen, wobei n eine ganze Zahl von 1 bis 4 sein kann und bevorzugt 1 oder 2 ist. wobei R¹ und R² eine Bindung oder einen (CH₂)ₘ-Rest, worin m 1, 2 oder 3 ist, bedeuten.verwendet wird.

6. Verwendung nach einem der vorhergehenden Ansprüche, **dadurch gekennzeichnet, dass** als vernetzendes Monomer eine der folgenden Verbindungen 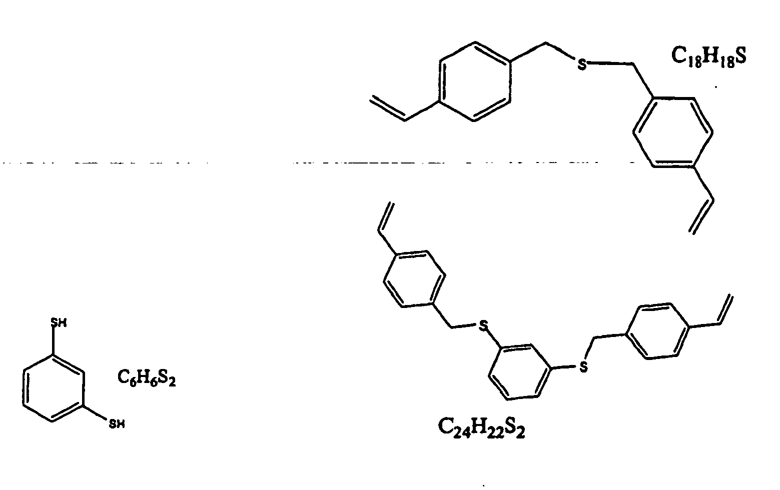 oder eine Mischung davon verwendet wird.

7. Verwendung nach einem der vorhergehenden Ansprüche, **dadurch gekennzeichnet, dass** das Monomer a) in einem Anteil von mindestens 30 Gew.-% vorhanden ist.

8. Verwendung nach einem der vorhergehenden Ansprüche, **dadurch gekennzeichnet, dass** das Polymer einen Brechungsindex von 1,60 oder mehr hat.

9. Verwendung einer Polymerzusammensetzung nach einem der Ansprüche 1 bis 8 als Augenimplantat, insbesondere Homhautimplantat.

10. Verwendung nach einem der Ansprüche 1 bis 9, **dadurch gekennzeichnet, dass** die Zusammensetzung für Kontaktlinsen, Keratoprothesen, Hornhautringe oder -inlays oder IOL verwendet wird.

## Claims

1. Use of a polymer composition prepared by polymerisation of at least a main monomer
a) having the formula 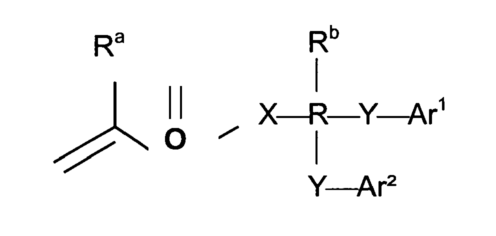 wherein X can be O or NR^{c},
Y can be O, S or NR^{c}, respectively,
R denotes a linear, branched or cyclic hydrocarbon residue having 1 to 6 carbon atoms,
R^{a} denotes hydrogen or a methyl residue,
R^{b} can denote hydrogen, a C₁-C₅ alkyl residue or Y-Ar³,
R^{c} denotes hydrogen, a linear branched or cyclic hydrocarbon residue having 1 to 6 carbon atoms or an aryl group,
Ar¹, Ar² and Ar³ denote each independently from each other, an aryl group being bonded to Y via a bond or via (-CH₂)ₙ, wherein n can be 0, 1, 2 or 3, and wherein the aryl group can be substituted with 1 to 4 substituents selected among C₁-C₅ alkyl, C₁-C₅ alkoxy, mono- and di-substituted amino, wherein the substituents can be selected among residues R^{c} as defined above,
b) a cross-linked monomer and
c) optionally further monomers for adjusting properties such as a refraction index, surface properties, a glass transition temperature, mechanical properties, UV absorption and/or for colouration,
for ophthalmologic devices.

2. Use of a polymer composition according to claim 1, wherein in the monomer a) Y denotes S.

3. Use of a polymer composition according to claim 1 or claim 2, wherein as the monomer a) one of the following compounds 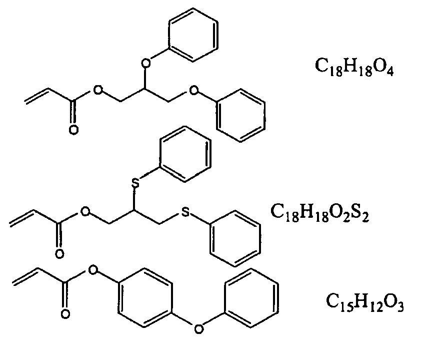 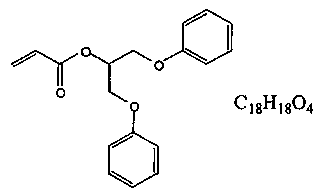 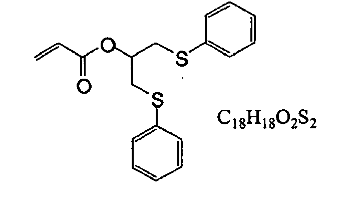 or a mixture thereof is used.

4. Use according to any one of the preceding claims, wherein POEMA or HEMA is employed as the monomer c).

5. A polymer composition according to any one of the preceding claims, **characterized in that** as the cross-linked monomer a compound with the following formula II 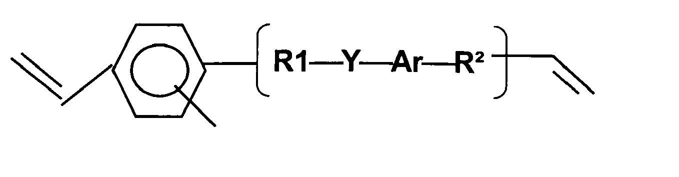 is used, which at both ends, respectively, carries a terminally unsaturated group, wherein Y can denote O or S, Ar is an aromatic residue, particularly a phenyl residue, which can be substituted with 0 or 1 to 4 substituents selected among C₁-C₅ alkyl residues, C₁-C₅ alkoxy residues and halogens, wherein n can be an integer from 1 to 4 and preferably is 1 or 2, wherein R¹ and R² denote a bond or a (CH₂)ₘ remainder, wherein m is 1, 2 or 3.

6. Use according to any one of the preceding claims, **characterized in that** as the cross-linked monomer one of the following compounds 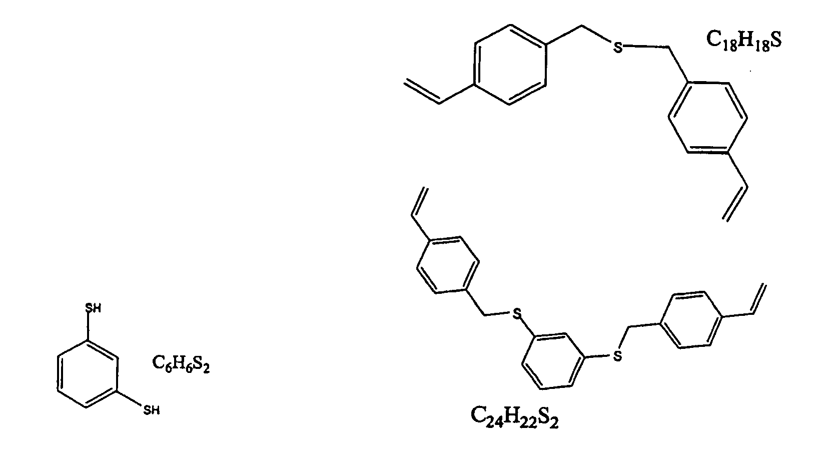 or a mixture thereof is used.

7. Use according to any one of the preceding claims, **characterized in that** the monomer a) is present in a proportion of at least 30 % by weight.

8. Use according to any one of the preceding claims, **characterized in that** the polymer has a refraction index of 1.60 or more.

9. Use of a polymer composition according to any one of the claims 1 to 8 as an eye implant, in particular a cornea implant.

10. Use according to any one of the claims 1 to 9, **characterized in that** the composition is used for contact lenses, keratoprostheses, cornea rings or inlays or IOLs.

## Revendications

1. Utilisation d'une composition de polymères obtenue par polymérisation d'au moins un monomère principal a) de formule 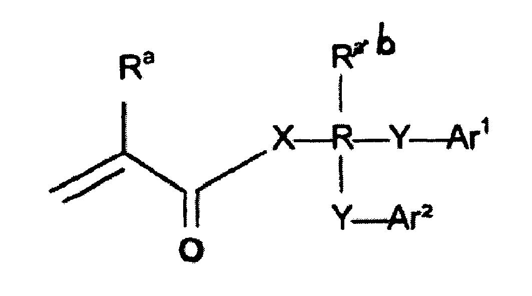 dans laquelle X peut être O ou NR^{c},
Y peut être à chaque fois O, S ou NR^{c},
R est un radical d'hydrocarbure linéaire, ramifié ou cyclique comportant 1 à 6 atomes de carbone,
R^{a} est un atome d'hydrogène ou un radical méthyle,
R^{b} est un atome d'hydrogène, un radical alkyle en C₁ à C₅ ou Y-Ar³,
R^{c} est un atome d'hydrogène, un radical d'hydrocarbure linéaire, ramifié ou cyclique comportant 1 à 6 atomes de carbone ou un groupe aryle,
Ar¹, Ar² et Ar³ sont à chaque fois, indépendamment les uns des autres, un groupe aryle qui est lié à Y par une liaison ou par (-CH₂)ₙ, où n peut être 0, 1, 2 ou 3, et le groupe aryle peut être substitué par 1 à 4 substituants, choisis parmi les groupes alkyle en C₁ à C₅, alcoxy en C₁ à C₅, amino mono- ou di-substitué, les substituants pouvant être choisis parmi les radicaux R^{c} tels que définis ci-dessus,
b) un monomère réticulé et
c) éventuellement d'autres monomères pour ajustement des propriétés telles que : indice de réfraction, propriétés de surface, température de transition vitreuse, propriétés de résistance, absorption d'UV et/ou coloration,
pour des dispositifs ophtalmologiques.

2. Utilisation d'une composition de polymères selon la revendication 1, dans laquelle dans le monomère a), Y est S.

3. Utilisation d'une composition de polymères selon la revendication 1 ou la revendication 2, dans laquelle on utilise comme monomère a), l'un des composés suivants 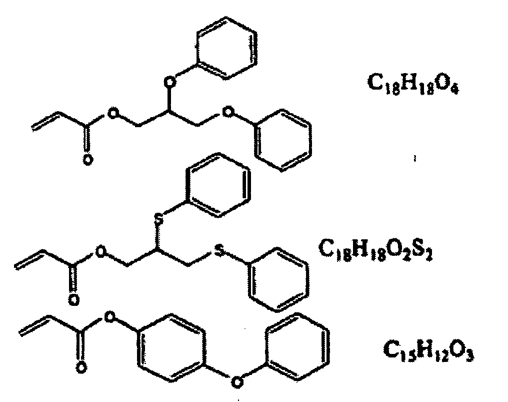 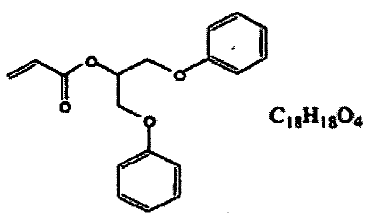 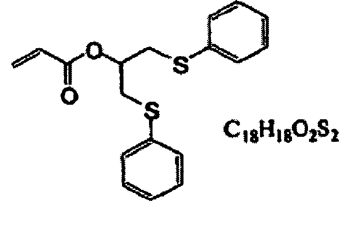 ou un mélange de ceux-ci.

4. Utilisation selon l'une des revendications précédentes, dans laquelle on utilise comme monomère c) du POEMA ou de l'HEMA.

5. Composition de polymères selon l'une des revendications précédentes, **caractérisée en ce que** l'on utilise comme monomère réticulé, un composé de formule II suivante 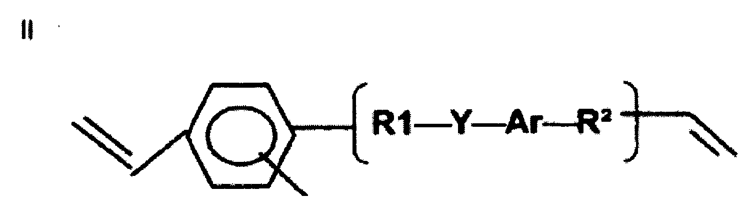 qui porte aux deux extrémités, respectivement, un groupe terminal insaturé, Y pouvant être O ou S, Ar, étant un radical aromatique, en particulier phényle, qui peut être substitué par 0 ou 1 à 4 substituants qui sont choisis parmi des radicaux alkyle en C₁ à C₅, des radicaux alcoxy en C₁ à C₅ et des atomes d'halogène, n pouvant être un nombre entier de 1 à 4 et étant de préférence 1 ou 2, R¹ et R² étant une liaison ou un radical (CH₂)ₘ, où m est 1, 2 ou 3.

6. Utilisation selon l'une des revendications précédentes, **caractérisée en ce que** l'on utilise comme monomère de réticulation, l'un des composés suivants 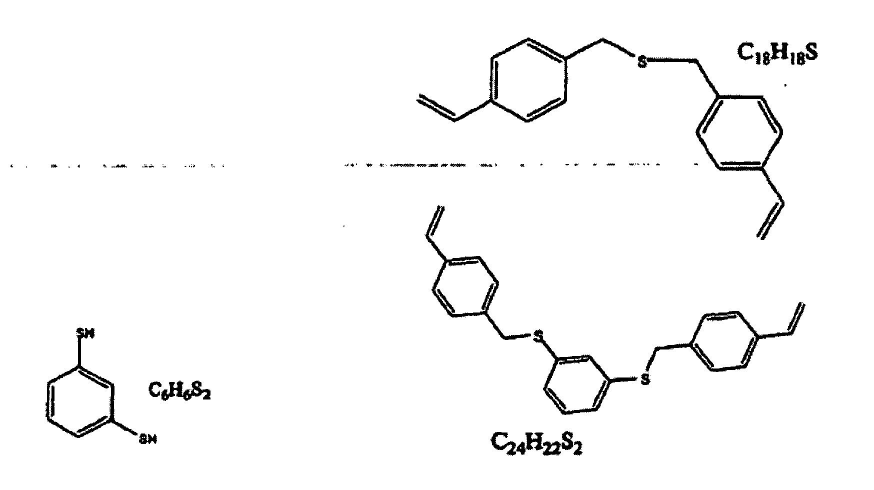 ou un mélange de ceux-ci.

7. Utilisation selon l'une des revendications précédentes, **caractérisée en ce que** le monomère a) est présent en une proportion d'au moins 30 % en poids.

8. Utilisation selon l'une des revendications précédentes, **caractérisée en ce que** le polymère a un indice de réfraction de 1,60 ou plus.

9. Utilisation d'une composition de polymères selon l'une des revendications 1 à 8, comme implant oculaire, en particulier, implant de cornée.

10. Utilisation selon l'une des revendications 1 à 9, **caractérisée en ce que** la composition est utilisée pour des lentilles de contact, des prothèses cornéennes, des anneaux cornéens ou des lentilles intra-cornéennes ou inlays ou des lentilles intra-oculaires.
